# EUROPÄISCHE PATENTSCHRIFT

(11) **EP 2 613 689 B1**
(45) Veröffentlichungstag und Bekanntmachung des Hinweises auf die Patenterteilung: **25.02.2015**
(21) Anmeldenummer: 11760439.7
(22) Anmeldetag: 08.09.2011
(51) Int. Cl.: A61B 5/00, A61B 6/14, G01B 11/25

(54) **DENTALE RÖNTGENEINRICHTUNG MIT BILDERFASSUNGSEINHEIT ZUR OBERFLÄCHENERFASSUNG UND VERFAHREN ZUR ERZEUGUNG EINER RÖNTGENAUFNAHME EINES PATIENTEN**
DENTAL X-RAY DEVICE WITH IMAGING UNIT FOR SURFACE IMAGING, AND METHOD FOR GENERATING AN X-RAY OF A PATIENT
DISPOSITIF DE RADIOGRAPHIE DENTAIRE COMPORTANT UNE UNITÉ DE DÉTECTION D'IMAGES POUR LA DÉTECTION DE SURFACE ET PROCÉDÉ DE RÉALISATION D'UNE RADIOGRAPHIE D'UN PATIENT

(30) Priorität: 20.09.2010 US 384476 P; 08.09.2010 DE 102010040386
(43) Veröffentlichungstag der Anmeldung: 17.07.2013
(73) Patentinhaber: Sirona Dental Systems GmbH, 64625 Bensheim (DE)
(72) Erfinder: LINDENBERG, Kai, 64395 Wersau (DE); BECKHAUS, Christian, 64297 Darmstadt (DE); ULRICI, Johannes, 64293 Darmstadt (DE)
(74) Vertreter: Sommer, Peter
(86) Internationale Anmeldenummer: PCT/EP2011/065587
(87) Internationale Veröffentlichungsnummer: WO 2012/032132

(56) Entgegenhaltungen:
- WO-A1-2011/076416
- WO-A1-2011/095694
- DE-A1-102005 022 540
- DE-A1-102008 022 922

## Beschreibung

### Technisches Gebiet

Die Erfindung betrifft eine dentale Röntgenvorrichtung zur Erzeugung einer Röntgenaufnahme eines Patienten, wobei die Röntgenvorrichtung zusätzlich eine Bilderfassungseinheit zur Oberflächenerfassung aufweist. Die Erfindung betrifft weiterhin ein Verfahren zur Erzeugung einer Röntgenaufnahme eines Patienten, wobei ein System aus Röntgenquelle und Röntgendetektor in einem Umlauf um den Patienten herum bewegt werden.

### Stand der Technik

Aus dem Stand der Technik sind mehrere Röntgenvorrichtungen mit zusätzlicher optischer Bilderfassungseinheit sowie Verfahren zum Betreiben solcher Vorrichtungen bekannt. Aus der DE 10 2004 020 370 B4 ist beispielsweise ein Röntgenstrahler mit einer im oder am Gehäuse angeordneten Bilderfassungseinrichtung für Lichtwellen im sichtbaren Bereich bekannt

Aus der DE 10 2008 035 412 A1 ist ein Röntgengerät mit einer am Röntgengerät angeordneten optische Kamera bekannt. Mit der Kamera wird eine Aufnahme eines im Röntgengerät positionierten Patienten erzeugt und zu einer bildliche Darstellung verarbeitet. Anhand dieser bildlichen Darstellung werden die Einstellungs- und/oder Steuerdaten zur Erstellung einer Röntgenaufnahme überprüft und verändert, bevor die Röntgenaufnahme erzeugt wird.

Die US 7,551,711 B2 beschreibt ebenfalls ein Röntgengerät mit einer am Röntgengerät angeordneten optischen Kamera, wobei die optischen Daten synchron zur Röntgenaufnahme erzeugt werden und ein aus den optischen Daten erzeugtes O-berflächenbild des Patienten zu besseren Interpretation der Röntgenaufnahmen bereitgestellt wird.

Aus der US 6,574,296 B2 ist ebenfalls ein Röntgengerät mit einer optischen Aufnahmevorrichtung bekannt, wobei die optischen Daten während der Erzeugung der Röntgenaufnahme ermittelt werden.

Aus der US 6,081,739 ist eine Röntgeneinrichtung mit einem Ultraschall- oder optischen Detektor und einem Farbvideodetektor zur gleichzeitigen Erzeugung einer Röntgenaufnahme, eines Höhenprofils und einer farbigen Videoaufnahme bekannt. Anstatt einen Ultraschall- oder optischen Detektor zur Ermittlung der Oberflächendaten zu verwenden, kann auch ein farbiger 3D-Scanner verwendet werden, der sowohl die Farbdaten als auch die Oberflächendaten ermittelt.

Ein Nachteil dieser Verfahren und Vorrichtungen ist, dass keine aktive Beleuchtung vorgesehen ist, weshalb Robustheit, Genauigkeit und Vollständigkeit der bildlichen Darstellung beeinträchtigt sind.

Aus der DE 10 2005 022 540 B4 ist ein Röntgengerät mit einem optischen Sensor bekannt, wobei die mit dem optischen Sensor erfasste Oberfläche bei der Rekonstruktion der mit dem Röntgengerät erzeugten Projektionsbilder zu einer 3D-Röntgenaufnahme zur Ergänzung von fehlenden Bilddaten verwendet. Der optische Sensor umfasst eine Lichtquelle und eine Kamera, wobei die Lichtquelle aus einer ersten Richtung ein Streifenmuster auf den Patienten projiziert und die Kamera das vom Patienten zurückgestreute Licht aus einer weiteren Richtung aufnimmt, so dass die Oberfläche nach dem Triangulationsverfahren ermittelt werden kann.

Auch aus der DE 103 17 137 A1 ist eine Röntgeneinrichtung mit einem zusätzlichen 3D-Sensor mit einer Lichtquelle zur Erzeugung eines Bilddatensatzes von zumindest einem Teil der Oberfläche des Patienten bekannt, wobei mittels eines schwenkbaren Umlenkspiegels das Licht der Lichtquelle linienförmig auf den Patienten projiziert wird und die Bilddaten synchron zur Erzeugung der Röntgenaufnahme aufgenommen werden.

Aus der DE 10 2008 022 922 A1 ist eine Röntgeneinrichtung sowie ein Verfahren zur Erstellung einer 3d Darstellung von einem Objekt bekannt, wobei die Röntgeneinheit zusätzlich eine Oberflächenabbildungseinheit aufweist, die beispielsweise an der Tragevorrichtung der Röntgeneinheit angeordnet sind und wobei ein Zentralstrahl für die Oberflächenaufnahme mit einem Zentralstrahl für die Röntgenaufnahme zur Deckung gebracht sind.

Ein Nachteil dieses Verfahren ist, dass keine Farb- oder Helligkeitsinformationen des Patienten ermittelt werden können. Auch die Erzeugung einer Lichtlinie mittels einer punktförmigen oder zumindest kleinflächigen Quelle und einer schwenkbaren Umlenkvorrichtung ist von Nachteil, da es zu Abschattungen bzw. nicht beleuchteten Bereichen der Linie auf der Patientenoberfläche kommen kann, was zu unvollständigen Datensätzen führt.

Die Aufgabe der vorliegenden Erfindung ist daher ein Verfahren zur robusten, genauen und synchronen Erfassung eines dentalen tomographischen Datensatzes eines Patienten und eines mit dem tomographischen Datensatz verknüpfbaren farbigen Höhenprofils des Patientengesichts mit offenen Augen sowie eine Vorrichtung hierfür bereitzustellen.

### Darstellung der Erfindung

Die Erfindung betrifft eine dentale Röntgeneinrichtung zur Erzeugung einer Röntgenaufnahme eines Patienten, umfassend eine Röntgenquelle und einen Röntgendetektor, die jeweils an einer Tragevorrichtung angeordnet sind und die mittels der Tragevorrichtung zumindest teilweise um einen sich zwischen der Röntgenquelle und dem Röntgendetektor befindlichen Aufnahmeraum herum bewegbar sind, sowie mindestens eine Bilderfassungseinheit zur Oberflächenerfassung eines im Aufnahmeraum positionierten Patienten. Die Bilderfassungseinheit weist mindestens eine Lichtquelle auf, die ein in den Aufnahmeraum gerichtetes polychromatisches Lichtbündel sichtbaren Lichts erzeugt. Die Bilderfassungseinheit weist weiterhin mindestens eine Kante auf, die zur Erzeugung einer Schattenkante im Aufnahmeraum zumindest teilweise zwischen der Lichtquelle und dem Aufnahmeraum in das Lichtbündel hineinragt. Weiterhin weist die Bilderfassungseinheit mindestens einen optischen Detektor auf. Die mindestens eine Lichtquelle, die mindestens eine Kante und der mindestens eine optische Detektor sind direkt an der Tragevorrichtung und/oder an der Röntgenquelle und/oder am Röntgendetektor angeordnet und zusammen mit der Röntgenquelle und dem Röntgendetektor um den Aufnahmeraum herum bewegbar. Die relativen Positionen der mindestens einen Lichtquelle, der mindestens einen Kante und des mindestens einen optischer Detektors zueinander sind bekannt.

Bei dem Aufnahmeraum handelt es sich um einen zwischen der Röntgenquelle und dem Röntgendetektor angeordneten Raum innerhalb der Röntgeneinrichtung, der zumindest die zu vermessenden Bereiche, also die Messvolumina für die Röntgenaufnahme und die Messbereiche für die Oberflächenvermessung und die Farbvermessung umfasst.

Die zur Röntgenaufnahme zusätzlich erzeugbaren optischen Daten, nämlich Oberflächendaten und Farbdaten, die durch das Bereitstellen einer Bilderfassungseinheit aufnehmbar sind, können die Orientierung und damit auch die Interpretation der Röntgenaufnahme erleichtern.

Durch die mindestens eine Kante, die in den in den Aufnahmeraum gerichteten Strahlengang hineinragt, bildet sich im Aufnahmeraum, beispielsweise auf dem Kopf eines dort positionierten Patienten ein heller und ein dunkler Bereich aus. Dadurch verläuft eine sich als Grenzlinie zwischen dem hellen und dem dunklen Bereich ausbildende Linie bzw. Schattenkante zumindest über Teile des Kopfs des Patienten aus. Je nach Ausrichtung der Kante kann diese Schattenkante beispielsweise in vertikaler Richtung über einen zu vermessenden Teil des Kopfs oder den gesamten Kopf des Patienten verlaufen.

Das Bereitstellen einer erfindungsgemäßen Bilderfassungseinheit mit einer Kante zur Erzeugung einer Schattenkante ermöglicht die Verwendung strukturierten Lichts für die Vermessung des Objekts. Unter strukturiertem Licht wird hier Licht verstanden, welches in seiner räumlichen Ausdehnung eine Struktur im Sinne von hellen und dunklen Bereichen aufweist. Dies kann beispielsweise ein Muster, ein Streifen oder eine Kante sein, die dem Licht quer zu seiner Ausbreitungsrichtung eine Struktur verleiht. Im Vergleich zu einer Vermessung mit unstrukturiertem Licht, also einer lediglich homogenen Ausleuchtung des zu vermessenden Objekts, ist eine Vermessung mit strukturiertem Licht in der Regel robuster und erfordert weniger Rechenzeit

Eine einzelne Schattenkante ist kein Spezialfall eines Gitters oder gar eines Projektors mit frei wählbarem Muster, wie es etwa als Ausführungsbeispiel in der DE 10 2008 022 922 A1 erwähnt wird. Es ist vielmehr von besonderer Bedeutung, dass ein definierter möglichst großer Bereich eines aufgenommenen Kamerarohbildes gleichmäßig ausgeleuchtet ist, also ohne starke Helligkeitssprünge wie bei einem Gitter. Dieser Bereich dient zur Ermittlung der Farbinformation des Objektes, ohne dass entweder die Lichtquelle oder das projizierte Muster oder eine Schattenkante ein- bzw. ausgeschaltet werden muss. Des weiteren kann dieser Bereich auch der Erhöhung der Geometriegenauigkeit in einem weiteren Analyseschritt durch Standardverfahren der Stereometrie dienen. Die Verwendung eines Gitters in den aufgenommenen Bildern würde die Qualität des Oberflächenmodells sowohl in der Genauigkeit der Geometrie als auch in der Farbtreue und Beleuchtungsunabhängigkeit der Farbinformation deutlich einschränken. Die Detektion der Schattenkante in der Aufnahme ist besonders zuverlässig und im Vergleich zu der Detektion eines Gitters oder eines sonstigen Musters einfach.

Das direkt von der Kante ausgehende, einen beleuchteten Bereich begrenzende Licht, das zur Schattenkante im Aufnahmeraum, beispielsweise zu einem zu vermessenden Objekt hin verläuft bzw. die Schattenkante bildet, wird im Folgenden als beleuchtungsseitiger Grenzstrahlengang bezeichnet.

Das direkt von der Schattenkante ausgehende, zum optischen Detektor verlaufenden Licht wird entsprechend als beobachtungsseitiger Grenzstrahlengang bezeichnet.

Durch die festen zueinander bekannten Positionen der mindestens einen Lichtquelle, der mindestens einen Kante und des mindestens einen optischen Detektors liegt ein fester Winkel zwischen dem beleuchtungsseitigen Grenzstrahlengang und dem beobachtungsseitigen Grenzstrahlengang vor. Aus diesem Winkel, der beispielsweise vermessen werden kann, können nach der Methode der Triangulation anhand des Verlaufs der Schattenkante Oberflächendaten berechnet werden, wobei die für die Berechnung notwendige Basislänge der Abstand von der Kamera zum Grenzstrahlengang ist.

Durch das Verwenden von polychromatischem Licht ist es möglich, mit der Aufnahme der vorab beschriebenen Bilderfassungseinheit zusätzlich Farbdaten zu erfassen. Es können hierfür eine oder mehrere polychromatische Lichtquellen, z.B. eine oder mehrere LEDs mit polychromatischem Emissionsspektrum, verwendet werden. Es ist auch möglich mehrere monochromatische Lichtquellen zur Erzeugung des einen polychromatischen Lichtbündels zu kombinieren.

Als optischer Detektor kann beispielsweise ein Farbsensor für die Erfassung der Farbdaten und ein weiterer Detektor zur Erfassung der Oberflächendaten vorgesehen werden, so können jeweils die optimalen Detektoreinstellungen für die Oberflächendatenermittlung und für die Farbdatenermittlung gewählt werden, die sich deutlich voneinander unterscheiden können.

Es ist auch möglich einen oder mehrere Farbsensoren zur Erfassung der Oberflächendaten und der Farbdaten zu verwenden, so dass beide Datensätze in demselben Koordinatensystem vermessen werden.

Werden mehrere Sensoren in dieser Weise verwendet, so werden insbesondere die im hellen Bereich neben der Schattenkante liegenden Punkte aus mehreren Winkeln erfasst. Durch die Redundanz der Punkte in den Bildern kann die Genauigkeit der Oberflächendaten durch einen zusätzlichen Vergleich unterschiedlicher Perspektiven deutlich erhöht werden. Hierfür können beispielsweise auf Parallaxe beruhende Verfahren wie die Stereokorrespondez als Beispiel eines stereometrischen Verfahrens verwendet werden.

Durch die feste Anordnung der Bilderzeugungseinheit am beweglichen Teil der Röntgeneinrichtung und das synchrone Aufnehmen kann eine Zuordnung der mit der Bilderfassungseinheit erzeugten optischen Daten zur Röntgenaufnahme hergestellt werden.

Des Weiteren gewährleistet diese feste Anordnung insbesondere der Lichtquelle und der Kante eine für einen im Aufnahmeraum positionierten Patienten vorhersehbare Änderung der Beleuchtung während einer Aufnahme, wodurch Irritationen und reflexartige Bewegungen des Patienten während der Aufnahme vermieden werden können.

Bei der Röntgeneinrichtung kann es sich beispielsweise um einen Orthopantomographen oder einen Computertomographen handeln, wobei es sich bei der mit der Röntgeneinrichtung erzeugten Röntgenaufnahme entsprechend um eine Schichtaufnahme oder auch um ein 3D-Röntgenbild handeln kann.

Vorteilhafterweise kann die Lichtquelle eine linear ausgedehnte Lichtleiste sein, die aus einer oder mehreren nebeneinander angeordneten Einzellichtquellen besteht.

Durch eine in vertikaler Richtung linear ausgedehnte Lichtquelle kann eine gleichmäßige und hinreichende Ausleuchtung des Patienten in vertikaler Richtung sichergestellt werden. So wird gewährleistet, dass sich die Schattenkante in vertikaler Richtung beispielsweise über das gesamte Gesicht eines im Aufnahmeraum positionierten Patienten deutlich ausbildet.

Die mindestens eine Lichtquelle kann beispielsweise eine maximale Beleuchtungsstärke von 5 lm/cm² aufweisen, wodurch das Vermessen eines Kopfes eines Patienten mit geöffneten Augen möglich ist, ohne dass weitere Schutzvorkehrungen für die Augen des Patienten vorgesehen werden müssen.

Vorteilhafterweise kann der optische Detektor sowohl Helligkeitsinformationen als auch Farbinformationen des Aufnahmeraums erfassen und als Aufnahme, auch als Kamerarohbild bezeichnet, bereitstellt.

Vorteilhafterweise kann in der Aufnahme ein Bereich von mehr als 50%, vorzugsweise mehr als 75% und insbesondere mehr als 85% frei von dem von der Schattenkante hervorgerufenen Schattenwurf sein. Dies hat den Vorteil, dass ein großer Bereich jeder Aufnahme für die Erfassung der winkelabhängigen Farbdaten sowie gegebenenfalls für die Anwendung stereometrischer Verfahren verwendet werden kann.

Die Erfindung betrifft weiterhin ein Verfahren zur Erzeugung einer Röntgenaufnahme eines Patienten mittels einer Röntgeneinrichtung, wobei mittels Röntgenstrahlung während eines zumindest teilweisen Umlaufs eines Systems aus einer Röntgenquelle und einem Röntgendetektor um einen sich zwischen der Röntgenquelle und dem Röntgendetektor befindlichen Aufnahmeraum von der Röntgenquelle erzeugte und den Aufnahmeraum durchlaufende Röntgenstrahlen aus mehreren verschiedenen Richtungen vom Detektor erfasst werden. Gleichzeitig mit der Erzeugung der Röntgenaufnahme während des zumindest teilweisen Umlaufs des Systems wird mittels einer sich mit dem System mitbewegenden Bilderfassungseinheit mit einem von mindestens einer Lichtquelle ausgehenden polychromatischen Lichtbündel mindestens eine Kante so in dem Aufnahmeraum abgebildet, dass sich ein heller Bereich und ein dunkler Bereich ausbilden, wobei der helle und der dunkle Bereich linienförmig aneinander angrenzen, so dass sich eine Schattenkante ausbildet, und dass zumindest Teile des hellen und des dunklen Bereichs und der Schattenkante mittels mindestens eines optischen Detektors aufgenommen und aus den Aufnahmen Farbdaten und Oberflächendaten ermittelt werden, wobei die mindestens eine Lichtquelle, die mindestens eine Kante und der mindestens eine optische Detektor zur Durchführung des Verfahrens an bekannten Positionen an der Röntgeneinrichtung angeordnet werden.

Bei der Röntgenaufnahme kann es sich beispielsweise um ein aus einzelnen Projektionsaufnahmen rekonstruiertes 3D-Röntgenbild oder auch um eine Schichtaufnahme handeln.

Die zusätzlichen Daten können die Orientierung und damit auch die Interpretation der Röntgenaufnahme erleichtern.

Durch das Projizieren einer Kante bilden sich auf dem Patienten ein heller und ein dunkler Bereich, wobei die Bereiche linienförmig aneinander angrenzen. Die dadurch gebildete Grenzlinie wird als Schattenkante bezeichnet. Der aufzunehmende Bereich des Patienten wird somit nicht homogen ausgeleuchtet, sondern es wird auf dem Patienten mit Licht eine Struktur, nämlich eine Schattenkante, erzeugt. Daher spricht man auch von der Verwendung strukturierten Lichts. Ein solches Verfahren ist in der Regel robuster und benötigt weniger Rechenleistung als Verfahren, die ohne strukturiertes Licht, also mit einer rein homogenen Ausleuchtung arbeiten.

Das Projizieren der Kante mit polychromatischem Licht ermöglicht es gleichzeitig sowohl Oberflächendaten als auch Farbdaten zu ermitteln.

Die Oberflächendaten können nach der Methode der Triangulation aus dem Verlauf der Schattenkante, die sich als linienförmige Grenze zwischen dem hellen und dem dunklen Bereich ausbildet und in den Aufnahmen der Bilderfassungseinheit erfasst wird, ermittelt werden. Dies ist insbesondere dadurch möglich, dass die Positionen der Lichtquelle, der Kante und des optischen Detektors bekannt sind, woraus sich Basislänge und Winkel für die Triangulation ermitteln lassen.

Durch die Projektion der Kante mit polychromatischem Licht enthält das von einem im Aufnahmeraum positionierten Patienten zurückgestreute Licht zu den Helligkeitsinformationen auch Farbinformationen des Patienten. Diese werden mit der Aufnahme der Bilderfassungseinheit zusätzlich erfasst. Hierfür kann beispielsweise ein Farbsensor als optischer Detektor verwendet werden. Es ist aber auch möglich beispielsweise einen Farbsensor zur Erfassung der Farbdaten und einen weiteren beispielsweise in einem engeren Wellenlängenbereich empfindlichen Sensor zur Erfassung der Schattenkante zu verwenden.

Durch die feste Anordnung der Bilderzeugungseinheit am beweglichen Teil der Röntgeneinrichtung und das synchrone Aufnehmen ist eine Zuordnung der mit der Bilderfassungseinheit erzeugten optischen Daten zur Röntgenaufnahme möglich.

Des Weiteren gewährleistet diese feste Anordnung insbesondere der Lichtquelle und der Kante, eine für einen im Aufnahmeraum positionierten Patienten vorhersehbare Änderung der Beleuchtung während eines Umlaufs des Systems, wodurch Irritationen und reflexartige Bewegungen des Patienten während der Aufnahme vermieden werden können.

Das Verfahren, das verwendet wird um ein farbiges Oberflächenmodell zu erhalten, nachdem die Daten aufgenommen wurden, ist mindestens zweistufig. In der ersten Stufe wird die Position der Schattenkante auf dem Kamerasensor für jedes Bild ermittelt und mit der etwa durch Kalibrierung bekannten Position des Sensors und der Beleuchtungseinheit in ein geometrisches Oberflächenmodell überführt. Im zweiten Schritt wird über alle Punkte, auch als Vertizes bezeichnet, des Oberflächenmodells iteriert und für jeden Punkt, auch als Vertex bezeichnet, die Farbinformation aus vielen Kamerarohbildern gespeichert. In jedem Kamerarohbild ist die Farbinformation eines Vertex für einen bestimmten Beleuchtungswinkel enthalten. In der Summe erhält man so eine winkelabhängige Farbinformation für jeden Punkt auf dem Oberflächenmodell.

Anders als bei der Verwendung einer Schattenkante würde ein Gitter die Winkelvielfalt sehr stark reduzieren und es stünden weniger Informationen zur Verfügung, um zum einen ein beleuchtungsunabhängiges Bild in hoher Qualität als auch zum anderen eine realistische künstliche Beleuchtung mithilfe der winkelabhängigen Farbdaten zu erzeugen.

Vorteilhafterweise können die Farbdaten und die Oberflächendaten aus den Aufnahmen ermittelt werden, die zu mehreren zeitlich aufeinanderfolgenden Zeitpunkten während des zumindest teilweisen Umlaufs des Systems erfasst wurden.

Je mehr Aufnahmen während des zumindest teilweisen Umlaufs des Systems mit der Bilderfassungseinheit erfasst werden, um so höher ist die Genauigkeit der Oberflächendaten und der Farbdaten. Des Weiteren können Bewegungen des Patienten in den optischen Daten erkannt werden.

Vorteilhafterweise können die Farbdaten und die Oberflächendaten aus den Aufnahmen ermittelt werden, die aus mindestens vier verschiedenen Richtungen erfasst wurden. Durch das Erfassen der Oberflächendaten und der Farbdaten aus möglichst vielen Richtungen ist es möglich, die Genauigkeit dieser Daten zu erhöhen.

Vorteilhafterweise können die Farbdaten und die Oberflächendaten aus den Aufnahmen ermittelt werden, die aus winkelmäßig über den zumindest teilweisen Umlauf des Systems gleich verteilten Richtungen erfasst wurden.

Durch die gleichmäßige Verteilung der Aufnahmen über den Winkel des Umlaufs ergibt sich aus den aufgenommenen Daten direkt ein gleichmäßiges Oberflächenmodell. Beispielsweise entspricht je einer aufgenommenen Schattenkante eine Zeile im Oberflächenmodell.

Vorteilhafterweise können Aufnahmen für die Farbdaten und Aufnahmen für die Oberflächendaten während des zumindest teilweisen Umlaufs nacheinander in gleichen zeitlichen Abständen erfasst werden.

Zeitlich gleich beabstandete Aufnahmen lassen sich technisch besonders leicht umsetzen, wobei zeitlich gleiche Abstände der Aufnahmen bei einer konstanten Winkelgeschwindigkeit des Umlaufs zu winkelmäßig über den Umlauf gleich verteilten Richtungen der Aufnahmen führt. Allerdings sind nicht alle Röntgenaufnahmen mit konstanter Winkelgeschwindigkeit erstellbar. So ist zu beachten, dass die Winkelgeschwindigkeit zur Erstellung einer Schichtaufnahme in der Regel nicht konstant ist.

Vorteilhafterweise kann vor einer Aufnahme oder einer Serie von Aufnahmen eine Röntgenaufnahme sowie Oberflächendaten und Farbdaten eines in der Röntgeneinrichtung vorhandenen oder zu diesem Zweck in die Röntgeneinrichtung eingebrachten Kalibrierkörpers erzeugt und die relative Position der Farbdaten und der Oberflächendaten zu der Röntgenaufnahme bestimmt werden.

Durch das Einbringen eines Kalibrierkörpers oder das verwenden eines im Aufnahmeraum vorhandenen Kalibrierkörpers ist es möglich, die relativen Positionen der Farbdaten und der Oberflächendaten zur Röntgenaufnahme auf einfache Weise zu bestimmen.

Hierfür geeignete Kalibrierkörper sind aus dem Stand der Technik bekannt.

Ein solcher Kalibierkörper kann beispielsweise als ein eigenständiger Körper ausgebildet sein, der nur zum Zweck der Kalibration innerhalb des Aufnahmeraums angeordnet wird, beispielsweise durch Anbringen an einem Teil der Röntgeneinrichtung. Der Kalibrierkörper kann auch ein an einem Teil der Röntgeneinrichtung im Aufnahmeraum fest angeordneter Körper sein. Es ist auch möglich, den Kalibrierkörper als ein auf einem Teil der Röntgeneinrichtung im Aufnahmeraum angeordnetes zweidimensionales Bild, beispielsweise als einen Aufdruck zu gestalten.

Die Farbdaten, die Oberflächendaten und die Röntgenaufnahme können beispielsweise anhand der relativen Position der Farbdaten und der Oberflächendaten zur Röntgenaufnahme mittels Transformation in ein gemeinsames Koordinatensystem überführt werden.

Dadurch ist es möglich, die Oberflächendaten und die Farbdaten mit der Röntgenaufnahme zu verknüpfen und beispielsweise die Orientierung in den Röntgendaten zu erleichtern. Handelt es sich bei der Röntgenaufnahme um eine Schichtaufnahme, so kann beispielsweise ein 3D-Koordinatensystem als gemeinsames Koordinatensystem dienen, wobei die Schichtaufnahme einen gebogenen Bereich in diesem Koordinatensystem einnimmt. Es ist beispielsweise auch möglich, das 2D-Koordinatensystem der Schichtaufnahme als gemeinsames Koordinatensystem auszuwählen.

Vorteilhafterweise können die Farbdaten und die Oberflächendaten des Patienten mit der Röntgenaufnahme gemeinsam dargestellt werden.

Es ist möglich die erfassten Daten in verschiedenen Fenstern nebeneinander anzuzeigen, beispielsweise eine Darstellung einer farbigen Oberfläche in einem Fenster und die Röntgenaufnahme in einem weiteren Fenster. Es ist auch möglich die Daten alle gemeinsam in einem Fenster darzustellen.

Die Farbdaten und die Oberflächendaten des Patienten und die Röntgenaufnahme können beispielsweise gleichzeitig in einem gemeinsamen Koordinatensystem angezeigt werden.

Dadurch wird der direkte Zusammenhang der Aufnahmen hergestellt und beispielsweise die Interpretation der Röntgenaufnahme erleichtert. Hierfür können die Daten beispielsweise so überlagert werden, dass beim Zoomen aus Sicht des Betrachters ein nahtloser Übergang zwischen dem einen und dem anderen Datensatz realisiert wird. Es wäre auch möglich, die Farb- und Oberflächendaten so transparent anzuzeigen, dass die im gleichen Koordinatensystem angezeigte Röntgenaufnahme hindurchscheint.

Vorteilhafterweise kann vor einer Aufnahme oder einer Serie von Aufnahmen eine Röntgenaufnahme sowie Oberflächendaten und Farbdaten eines in der Röntgeneinrichtung vorhandenen oder zu diesem Zweck in die Röntgeneinrichtung eingebrachten Kalibrierkörpers erzeugt und eine Farbkorrektur bestimmt werden.

Durch ein solches Kalibrieren des Systems hinsichtlich der Farbe kann sichergestellt werden, dass die Aufnahmen keinen Farbstich erhalten. Hierzu wird mit der Farbkorrektur insbesondere die Umgebungsbeleuchtung berücksichtigt. Für eine solche Farbkalibration geeignete Kalibrierkörper sind aus dem Stand der Technik bekannt.

Vorteilhafterweise kann aus mindestens zwei Aufnahmen mittels eines stereometrischen Verfahrens ein geometrisches Modell der Oberfläche erzeugt werden, wobei die Oberflächendaten bei der Durchführung des stereometrischen Verfahrens verwendet werden.

Die geometrische Auflösung des Oberflächenmodells kann verbessert werden, indem die Aufnahmen, vorzugsweise nur der beleuchtete Bereich jeder der Aufnahmen, für stereometrische Zwecke verwendet werden. Bei stereometrischen Verfahren wird die Position eines Punktes des aufgenommenen Objektes in zwei Bildern identifiziert und mithilfe der bekannten Position der Kamerasensoren auf die genaue Lage im Raum zurückgeführt. Die bei stereometrischen Verfahren vorhandene lange Laufzeit der Algorithmen kann deutlich verkürzt werden, indem das bereits mithilfe der Schattenkante ermittelte geometrische Modell als Startwert für die Optimierung des stereometrischen Verfahrens verwendet wird.

Vorteilhafterweise kann die Auflösung des mittels des stereometrischen Verfahrens erzeugten geometrisches Modells der Oberfläche höher sein als die Auflösung der Oberflächendaten.

Durch die vielen Aufnahmen, auch als Kamerarohbilder bezeichnet, steht eine große Menge an Informationen zur Verfügung, um die geometrische Genauigkeit gegenüber dem Oberflächenmodell um ein Vielfaches zu erhöhen.

### Kurzbeschreibung der Zeichnungen

Die Erfindung wird anhand der Zeichnungen erläutert. Es zeigt, die
- Fig. 1: eine erfindungsgemäße dentale Röntgeneinrichtung, die
- Fig. 2A, B: einen horizontalen Schnitt entlang der Linie AA aus Fig. 1 und einen in der Röntgeneinrichtung positionierten Kalibrierkörper, die
- Fig. 3A, B: das Prinzip der Projektion einer Schattenkante, die
- Fig. 4A, B: eine schematische Darstellung der ermittelten Daten.

### Ausführungsbeispiel

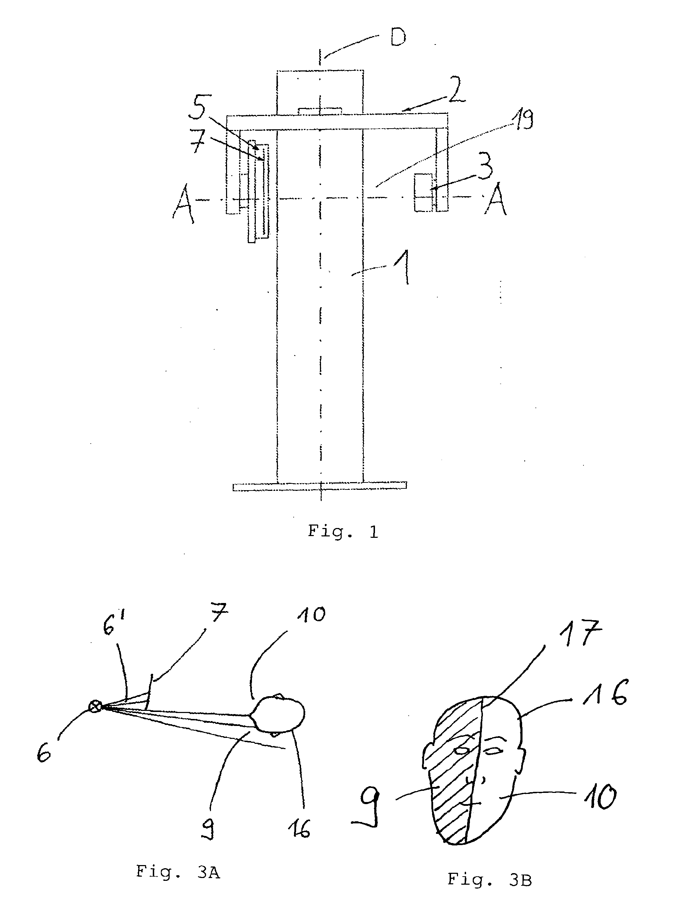

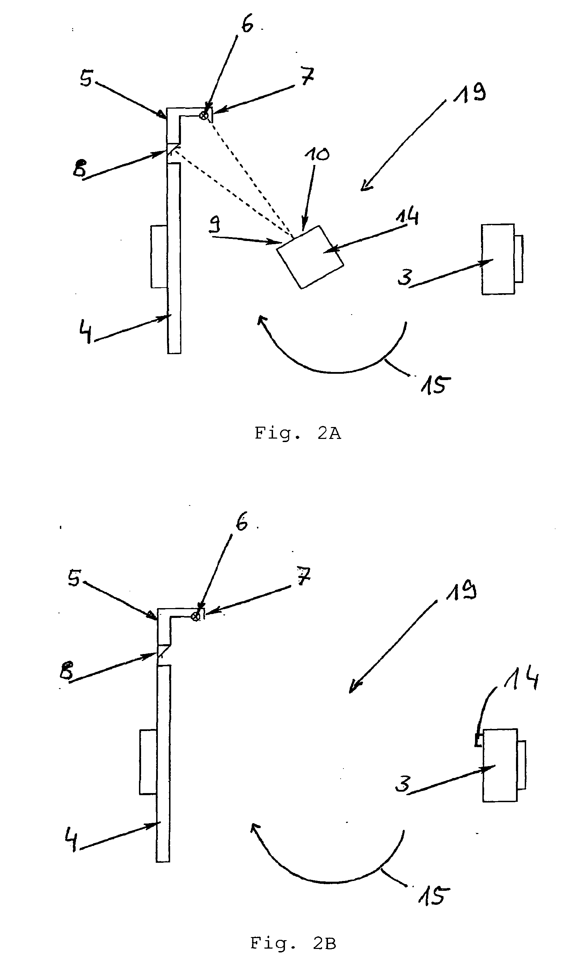

Die Fig. 1 zeigt eine seitliche Ansicht einer erfindungsgemäßen dentale Röntgeneinrichtung und Fig. 2A einen Schnitt dieser dentalen Röntgeneinrichtung entlang der Linie AA.

Die erfindungsgemäße Röntgeneinrichtung weist eine Halterung 1 auf, die beispielsweise, wie in Fig. 1 dargestellt, als Säule ausgebildet ist. Die Halterung könnte auch als eine Wandhalterung ausgebildet sein, beispielsweise ein horizontal verlaufender, an einer Wand angebrachter Arm.

An der Halterung 1 ist eine Tragevorrichtung 2 beweglich angebracht. Diese ist als horizontaler Arm ausgebildet, der in einer horizontalen Ebene um eine Drehachse D drehbar an einer Halterung 1 befestigt ist. Die Tragevorrichtung 2 wird im Folgenden auch als Tragarm 2 bezeichnet.

An dem drehbaren Tragarm 2 sind eine Röntgenquelle 3 und ein Röntgendetektor 4 so angeordnet, dass sie zueinander beabstandet sich gegenüberstehen und der Strahlengang der Röntgenstrahlen von der Röntgenquelle 3 zum Röntgendetektor 4 verläuft. Zwischen der Röntgenquelle 3 und dem Röntgendetektor 4 befindet sich ein Aufnahmeraum 19. Der Aufnahmeraum 19 umfasst zumindest die Aufnahmebereiche der Vermessungseinheiten, also ein Aufnahmevolumen, welches mittels der Röntgenquelle und dem Röntgendetektor aufnehmbar ist und einen Aufnahmebereich, der mittels der Bilderfassungseinheit aufnehmbar ist. Ein Patient 16 wird zur Vermessung so positioniert, dass sich ein zu vermessender Bereich, beispielsweise ein Teil des Kopfes, in den Aufnahmebereichen im Aufnahmeraum 19 befindet.

Am Röntgendetektor 4 ist weiterhin eine Bilderfassungseinheit 5 angeordnet, die aus mindestens einer Lichtquelle 6, mindestens einer Kante 7 und mindestens einem optischen Detektor 8 besteht.

Die mindestens eine Lichtquelle 6 zur Erzeugung eines polychromatischen Lichtbündels 6' und die mindestens eine Kante 7 sind so positioniert, dass die mindestens eine Kante 7 zumindest teilweise so in den Strahlengang des von der mindestens einen Lichtquelle 6 ausgehenden und auf den Patienten 16 gerichteten polychromatischen Lichtbündels 6' hineinragt und sich ein durch die Kante abgeschatteter dunkler Bereich 10 und ein vom Licht der Lichtquelle beleuchteter heller Bereich 9 auf dem im Aufnahmeraum 19 positionierten Patienten 16 ausbildet, wie es in den Fig. 3A und 3B dargestellt ist. Dabei bildet sich insbesondere eine Linie 17 als Grenze zwischen dem helleren Bereich 9 und dem dunkleren Bereich 10 aus. Diese Linie 17 wird auch als Schattenkante 17 bezeichnet.

Der mindestens eine optische Detektor 8 ist so positioniert, dass er zumindest diese sich auf dem Patienten 16 ausbildende Linie bzw. Schattenkante 17 erfasst.

Zur Erzeugung einer Röntgenaufnahme 13 eines im Aufnahmeraum 19 der dentalen Röntgeneinrichtung positionierten Patienten 16 wird der Tragarm 2 gedreht, so dass sich der Röntgendetektor 4 und die Röntgenquelle 3 entlang einer Umlaufrichtung 15 um den Patienten 16 herum bewegen, wobei der Röntgendetektor 4 während der Drehung die von der Röntgenquelle 3 ausgehenden und zumindest teilweise den Patienten 16 durchleuchtenden Röntgenstrahlen registriert.

Synchron zur Erzeugung der Röntgenaufnahme 13 werden während dieser Drehung des Tragarms 2 mittels der mindestens einen Lichtquelle 6, der mindestens einen Kante 7 und des mindestens einen optischen Detektors 8 die Oberflächendaten 11 und die Farbdaten 12 des Patienten 16 erzeugt, indem die Position der mittels der Lichtquelle 6 und der Kante 7 auf den Patienten 1 projizierten Schattenkante 17 durch das Drehen des Tragarms 2 während eines zumindest teilweisen Umlaufs des Systems relativ zum Patienten bewegt und die Schattenkante 17 in zumindest in einigen verschiedenen Positionen relativ zum Patienten 16 vom optischen Detektor 8 aufgenommen wird.

Die Oberflächendaten 11 werden aus den mit der Bilderfassungseinheit 5 erzeugten Aufnahmen nach der Methode der Triangulation anhand des Verlaufs der auf dem Patienten 16 erzeugten Schattenkante 17 ermittelt. Hierzu sind die festen Positionen der mindestens einen Lichtquelle 6, der mindestens einen Kante 7 und des mindestens einen optischen Detektors 8 und damit der daraus resultierenden festen Winkel bekannt.

Um zusätzlich Farbinformationen des Patienten 16 zu erhalten, erzeugt die mindestens eine Lichtquelle 6 polychromatisches Licht, nämlich das polychromatische Lichtbündel 6', welches von mindestens einem optischen Detektor 8, beispielsweise einem Farbsensor, farbsensitiv erfasst wird. Hierfür kann zusätzlich zu dem mindestens einen optischen Detektor 8 zur Erfassung der Schattenkante 17 und damit der Oberflächendaten 11 mindestens ein weiterer optischer Detektor 8 vorgesehen werden, der zur Erfassung der Farbdaten 12 verwendet wird. Es ist auch möglich einen oder mehrere optische Detektoren 8, beispielsweise Farbsensoren, jeweils zur Erfassung der Oberflächendaten 11 und der Farbdaten 12 zu verwenden.

Als Lichtquelle 6 können LEDs verwendet werden, beispielsweise mehrere in einer vertikalen Linie angeordneter LEDs. Es können als Lichtquelle 6 eine oder mehrere Lichtquellen mit einem polychromatischen Emissionsspektrum verwendet werde. Als Lichtquelle 6 können auch mehrere monochromatische Lichtquellen dienen, die erst gemeinsam das polychromatische Lichtbündel 6' bereitstellen.

Die Beleuchtungsstärke der Lichtquelle 6 ist so gewählt, dass der Patient 16 die Augen während der Durchführung einer Aufnahme geöffnet halten kann. Hierdurch sind keine weiteren Sicherheitsvorkehrungen notwendig.

Es hat sich gezeigt, dass schon mit einer Beleuchtungsstärke der Lichtquelle 6 von 30% des Umgebungslichts Oberflächendaten 11 und Farbdaten 12 mit einer ausreichenden Qualität ermittelbar sind. Eine gute Qualität der Oberflächendaten 11 und der Farbdaten 12 erreicht man insbesondere mit einer Beleuchtungsstärke der Lichtquelle 6, die 10 bis 100 Mal stärker als das Umgebungslicht ist. Von der Beleuchtungsstärke hängen die Qualität die Robustheit und die mögliche Geschwindigkeit der Aufnahmen wesentlich ab.

Die Lichtquelle 6, die Kante 7 und der optische Detektor 8 können auch an der Röntgenquelle 3 oder direkt am Tragarm 2 angeordnet werden. Durch all diese Anordnungen wird sichergestellt, dass sich die Lichtquelle 6, die Kante 7 und der optische Detektor 8 der Bilderfassungseinheit 5 bei einem zumindest teilweisen Umlauf des Systems zusammen mit diesem mitbewegen.

Dadurch wird ihre Bewegung, insbesondere die Bewegung der Lichtquelle 6 vorhersehbar. Dies erleichtert es einem Patienten 16 während einer Aufnahme, also während eines zumindest teilweisen Umlaufs des Systems ruhig zu halten und auch die Augen möglichst wenig zu bewegen. Irritationen und reflexartige Bewegungen des Patienten 16 während der Aufnahme, die zu deutlichen Qualitätseinbußen einer Röntgenaufnahme 13 führen würden, können so vermieden werden.

Fig. 2A zeigt weiterhin einen in den Aufnahmeraum 19 eingebrachten Kalibrierkörper 14. Der Kalibrierkörper 14 kann auch fest im Aufnahmeraum 19 der Röntgeneinrichtung angeordnet sein. Der Kalibrierkörper 14 kann beispielsweise an einem die Röntgenquelle 3 enthaltenden Gehäuseteil fest angebracht sein, wie dies in Fig. 2B skizziert ist.

Der Kalibrierkörper 14 kann dazu geeignet sein, die relative Position der Oberflächendaten 11 und der Farbdaten 12 zu der Röntgenaufnahme 13 zu bestimmen.

Der Kalibrierkörper 14 könnte auch dazu geeignet sein, die Positionen der Lichtquelle 6, der Kante 7 und des optischen Detektors 8 relativ zum Röntgengerät zu bestimmen.

Weiterhin kann es sich bei dem dargestellten Kalibrierkörper 14 um einen Kalibrierkörper handeln, der dazu verwendet werden kann, die Eigenschaften der Lichtquelle 6, beispielsweise deren Position beim Umlauf, deren Helligkeitsund Farbverteilung, die Eigenschaften der Kante 7, beispielsweise deren Position und Form, und die Eigenschaften des optischen Detektors 8, beispielsweise dessen Position, Farb- und Intensitätsempfindlichkeit und dessen Verzerrung, zu bestimmen.

Es ist auch möglich, einen Kalibrierkörper 14 zu verwenden, um die Eigenschaften der Röntgenquelle und des Röntgendetektors zu bestimmen.

Aus dem Stand der Technik sind Kalibrierkörper bekannt, die dazu geeignet sind jeweils einzelne oder mehrere der vorgenannten Parameter zu bestimmen.

Anhand dieser relativen Positionen ist eine Kombination der mit dem Röntgendetektor 4 aufgenommenen Röntgenaufnahmen 13 mit den mit der Bilderfassungseinheit 5 aufgenommenen Oberflächendaten 11 und Farbdaten 12 möglich. Es kann beispielsweise eine Transformationsmatrix bestimmt werden, die das Koordinatensystem der Röntgenaufnahme 13 mit dem Koordinatensystem der Oberflächendaten 11 und der Farbdaten 12 miteinander verbindet. Alle Daten können dann auch in ein gemeinsames Koordinatensystem überführt werden.

Dadurch kann die Röntgenaufnahme 13 gemeinsam mit den Oberflächendaten 11 und den Farbdaten 12 auf einer Anzeigevorrichtung 18, beispielsweise einem Monitor, gemeinsam angezeigt werden. Dies kann die Interpretation der Röntgenaufnahme 13 erleichtern.

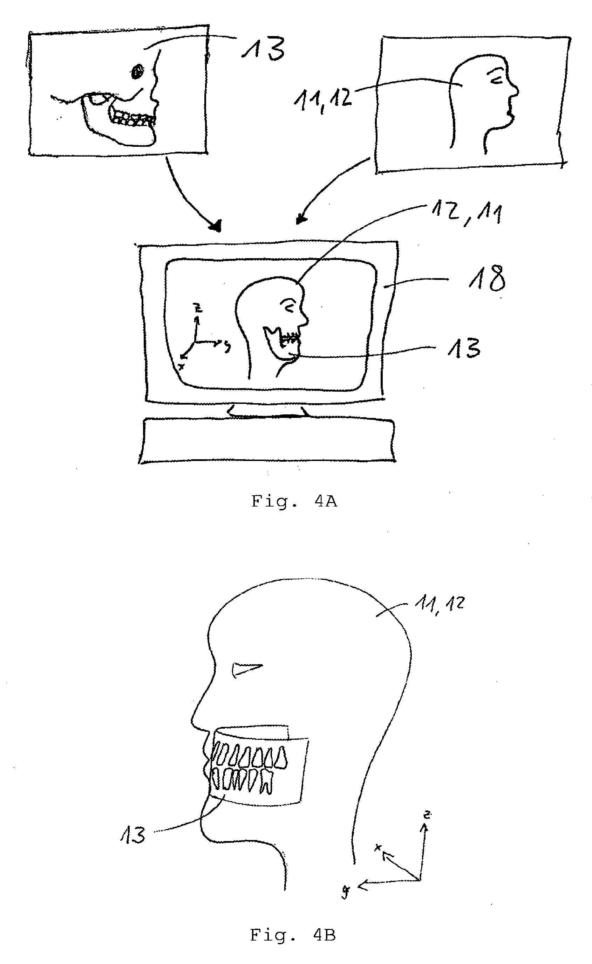

In Fig. 4A ist beispielhaft dargestellt, wie Farb- und Oberflächendaten 12, 11 in einem gemeinsamen Bild mit einer dreidimensionalen Röntgenaufnahme 13 auf einem Monitor dargestellt werden, wobei die Farb- und Oberflächendaten 12, 11 transparent dargestellt sind, so dass gleichzeitig auch die Röntgenaufnahme 13 erkennbar ist.

Fig. 4B zeigt schematisch, wie eine zweidimensionale Röntgenaufnahme 13, beispielsweise eine Panorama-Schichtaufnahme, innerhalb der Oberflächen- und Farbdaten 11, 12 dargestellt werden kann, wobei die Farb- und Oberflächendaten 12, 11 transparent dargestellt sind.

Es wäre auch möglich, das zweidimensionale Koordinatensystem einer Schichtaufnahme als gemeinsames Koordinatensystem auszuwählen und die Oberflächendaten 11 und die Farbdaten 12 in dieses zweidimensionale Koordinatensystem zu übertragen, beispielweise durch Projektion, um sie gemeinsam mit der als Schichtaufnahme ausgebildeten Röntgenaufnahme 13 dazustellen.

### Bezugszeichen

- 1: Halterung
- 2: Tragevorrichtung/Tragarm
- 3: Röntgenquelle
- 4: Röntgendetektor
- 5: Bilderfassungseinheit
- 6: Lichtquelle
- 6': Lichtbündel
- 7: Kante
- 8: Optischer Detektor
- 9: Heller Bereich auf dem Patient
- 10: Dunkler Bereich auf dem Patient
- 11: Oberflächendaten
- 12: Farbdaten
- 13: Röntgenaufnahme
- 14: Kalibrierkörper
- 15: Umlaufrichtung
- 16: Patient
- 17: Schattenkante, Linie zwischen hellen und dunklem Bereich
- 18: Anzeigevorrichtung
- 19: Aufnahmeraum
- D: Drehachse

## Patentansprüche

1. Dentale Röntgeneinrichtung zur Erzeugung einer Röntgenaufnahme (13) eines Patienten (16), umfassend eine Röntgenquelle (3) und einen Röntgendetektor (4), die jeweils an einer Tragevorrichtung (2) angeordnet sind und die mittels der Tragevorrichtung (2) zumindest teilweise um einen sich zwischen der Röntgenquelle (3) und dem Röntgendetektor (4) befindlichen Aufnahmeraum (19) herum bewegbar sind, sowie mindestens eine Bilderfassungseinheit (5) zur Oberflächenerfassung eines im Aufnahmeraum (19) positionierten Patienten (16), wobei die Bilderfassungseinheit mindestens eine Lichtquelle (6), die ein in den Aufnahmeraum (19) gerichtetes polychromatisches Lichtbündel (6') sichtbaren Lichts erzeugt und mindestens einen optischen Detektor (8) aufweist, wobei die mindestens eine Lichtquelle (6) und der mindestens eine optische Detektor (8) direkt an der Tragevorrichtung (2) und/oder an der Röntgenquelle (3) und/oder am Röntgendetektor (4) angeordnet und zusammen mit der Röntgenquelle (3) und dem Röntgendetektor (4) um den Aufnahmeraum (19) herum bewegbar sind, **dadurch gekennzeichnet, dass** die Bilderfassungseinheit (5) mindestens eine Kante (7) aufweist, die zur Erzeugung einer Schattenkante (17) im Aufnahmeraum (19) zumindest teilweise zwischen der Lichtquelle (6) und dem Aufnahmeraum (19) in das polychromatische Lichtbündel (6') hineinragt, wobei die mindestens eine Kante (7) direkt an der Tragevorrichtung (2) und/oder an der Röntgenquelle (3) und/oder am Röntgendetektor (4) angeordnet und zusammen mit der Röntgenquelle (3) und dem Röntgendetektor (4) um den Aufnahmeraum (19) herum bewegbar ist und wobei die relativen Positionen der mindestens einen Lichtquelle (6), der mindestens einen Kante (7) und des mindestens einen optischen Detektors (8) zueinander bekannt sind.

2. Dentale Röntgeneinrichtung nach Anspruch 1, **dadurch gekennzeichnet, dass** die Lichtquelle (6) eine linear ausgedehnte Lichtleiste ist, die aus einer oder mehreren nebeneinander angeordneten Einzellichtquellen besteht.

3. Dentale Röntgeneinrichtung nach einem der Ansprüche 1 oder 2, **dadurch gekennzeichnet, dass** der optische Detektor sowohl Helligkeitsinformationen als auch Farbinformationen des Aufnahmeraums (19) erfasst und als Aufnahme bereitstellt.

4. Dentale Röntgeneinrichtung nach Anspruch 3, **dadurch gekennzeichnet, dass** im Kamerarohbild ein Bereich von mehr als 50%, vorzugsweise mehr als 75% und insbesondere mehr als 85% frei von dem von der Schattenkante hervorgerufenen Schattenwurf ist.

5. Verfahren zur Erzeugung einer Röntgenaufnahme (13) eines Patienten (16) mittels einer Röntgeneinrichtung, wobei mittels Röntgenstrahlung während eines zumindest teilweisen Umlaufs eines Systems aus einer Röntgenquelle (3) und einem Röntgendetektor (4) um einen sich zwischen der Röntgenquelle (3) und dem Röntgendetektor (4) befindlichen Aufnahmeraum (19) von der Röntgenquelle (3) erzeugte und den Aufnahmeraum (19) durchlaufende Röntgenstrahlen aus mehreren verschiedenen Richtungen vom Röntgendetektor erfasst werden, **dadurch gekennzeichnet, dass** gleichzeitig mit der Erzeugung der Röntgenaufnahme (13) während des zumindest teilweisen Umlaufs des Systems mittels einer sich mit dem System mitbewegenden Bilderfassungseinheit (5) mit einem von mindestens einer Lichtquelle (6) ausgehenden polychromatischen Lichtbündel (6') mindestens eine Kante (7) so in dem Aufnahmeraum (19) abgebildet wird, dass sich ein heller Bereich (9) und ein dunkler Bereich (10) in dem Aufnahmeraum (19) ausbilden, wobei der helle und der dunkle Bereich (9, 10) linienförmig aneinander angrenzen, so dass sich eine Schattenkante (17) ausbildet, und dass zumindest Teile des hellen und des dunklen Bereichs (9, 10) und der Schattenkante (17) mittels mindestens eines optischen Detektors (8) aufgenommen und aus den Aufnahmen Farbdaten (12) und Oberflächendaten (11) ermittelt werden, wobei die mindestens eine Lichtquelle (6), die mindestens eine Kante (7) und der mindestens eine optische Detektor (8) zur Durchführung des Verfahrens an bekannten Positionen an der Röntgeneinrichtung angeordnet werden.

6. Verfahren nach Anspruch 5, **dadurch gekennzeichnet, dass** die Farbdaten (12) und die Oberflächendaten (11) aus den Aufnahmen ermittelt werden, die zu mehreren zeitlich aufeinanderfolgenden Zeitpunkten während des zumindest teilweisen Umlaufs des Systems erfasst wurden.

7. Verfahren nach Anspruch 5 oder 6, **dadurch gekennzeichnet, dass** die Farbdaten (12) und die Oberflächendaten (11) aus den Aufnahmen ermittelt werden, die aus mindestens vier verschiedenen Richtungen erfasst wurden.

8. Verfahren nach einem der Ansprüche 5 bis 7, **dadurch gekennzeichnet, dass** die Farbdaten (12) und die Oberflächendaten (11) aus den Aufnahmen ermittelt werden, die aus winkelmäßig über den zumindest teilweisen Umlauf des Systems gleich verteilten Richtungen erfasst wurden.

9. Verfahren nach einem der Ansprüche 5 bis 8, **dadurch gekennzeichnet, dass** Aufnahmen für die Farbdaten (12) und Aufnahmen für die Oberflächendaten (11) während des zumindest teilweisen Umlaufs nacheinander in gleichen zeitlichen Abständen erfasst werden.

10. Verfahren nach einem der Ansprüche 5 bis 9, **dadurch gekennzeichnet, dass** vor einer Aufnahme oder einer Serie von Aufnahmen eine Röntgenaufnahme (13) sowie Oberflächendaten (11) und Farbdaten (12) eines in dem Aufnahneraum (19) der Röntgeneinrichtung vorhandenen oder zu diesem Zweck in den Aufnahmeraum (19) der Röntgeneinrichtung eingebrachten Kalibrierkörpers (14) erzeugt werden und die relative Position der Farbdaten (12) und der Oberflächendaten (11) zu der Röntgenaufnahme (13) bestimmt wird.

11. Verfahren nach Anspruch 10, **dadurch gekennzeichnet, dass** die Farbdaten (12) und die Oberflächendaten (11) mit der Röntgenaufnahme (13) gemeinsam dargestellt werden.

12. Verfahren nach einem der Ansprüche 5 bis 11, **dadurch gekennzeichnet, dass** vor einer Aufnahme oder einer Serie von Aufnahmen eine Röntgenaufnahme (13) sowie Oberflächendaten (11) und Farbdaten (12) eines im Aufnahmeraum (19) der Röntgeneinrichtung vorhandenen oder zu diesem Zweck in den Aufnahmeraum (19) der Röntgeneinrichtung eingebrachten Kalibrierkörpers (14) erzeugt werden und eine Farbkorrektur bestimmt wird.

13. Verfahren nach einem der Ansprüche 5 bis 12, **dadurch gekennzeichnet, dass** aus mindestens zwei Aufnahmen mittels eines stereometrischen Verfahrens ein geometrisches Modell der Oberfläche erzeugt wird, wobei die Oberflächendaten bei der Durchführung des stereometrischen Verfahrens verwendet werden.

14. Verfahren nach Anspruch 13, **dadurch gekennzeichnet, dass** die Auflösung des mittels des stereometrischen Verfahrens erzeugten geometrisches Modells der Oberfläche höher ist als die Auflösung der Oberflächendaten.

15. Verfahren nach Anspruch 13 oder 14, **dadurch gekennzeichnet, dass** aus den Aufnahmen nur der beleuchtete Bereich für das stereometrische Verfahren verwendet wird.

## Claims

1. A dental x-ray device for generating a radiograph (13) of a patient (16) comprising an x-ray source (3) and an x-ray detector (4) which are each disposed on a support device (2) and can be moved at least partially by means of the support device (2) around an imaging area (19) located between the x-ray source (3) and the x-ray detector (4), as well as at least one imaging unit (5) for detecting the surface of a patient (16) positioned in the imaging area (19), the imaging unit having at least one light source (6) which generates a polychromatic light bundle (6') of visible light directed into the imaging area (6') and having at least one optical detector (8), the at least one light source (6) and the at least one optical detector (8) being disposed directly on the support device (2), and/or on the x-ray source (3), and/or on the x-ray detector (4), and being movable around the imaging area (19) together with the x-ray source (3) and the x-ray detector (4), **characterized in that** the imaging unit (5) has a least one edge (7) that extends at least partially between the light source (6) and the imaging area (19) into the polychromatic light bundle (6') to generate a shade edge (17) in the imaging area (19), the at least one edge (7) being disposed directly on the support device (2), and/or on the x-ray source (3), and/or on the x-ray detector (4), and being movable around the imaging area (19) together with the x-ray source (3) and x-ray detector (4), and the relative positions of the at least one light source (6), the at least one edge (7) and the at least one optical detector (8) relative to each other being known.

2. The dental x-ray device according to claim 1, **characterized in that** the light source (6) is a linearly extending light strip that consists of one or more adjacently disposed individual light sources.

3. The dental x-ray device according to one of claims 1 or 2, **characterized in that** the optical detector detects both brightness information as well as color information from the imaging area (19) and provides it as an image.

4. The dental imaging device according to claim 3, **characterized in that** an area of more than 50%, preferably more than 75% and in particular more than 85%, is free of the shade edge generated by the shadow.

5. A method to generate a radiograph (13) of a patient (16) by means of an x-ray device, x-rays generated by the x-ray source (3) and passing through the imaging area (19) from several different directions being detected by the x-ray detector by means of roentgen radiation during at least one at least partial revolution of a system consisting of an x-ray source (3) and an x-ray detector (4) around an imaging area (19) located between the x-ray source (3) and the x-ray detector (4), **characterized in that**, simultaneous to the generation of the radiograph (13) during the at least partial revolution of the system, at least one edge (7) is imaged in the imaging area (19) by means of an imaging unit (5) moving with the system with a polychromatic light bundle (6') emitted by at least one light source (6) such that a light area (9) and a dark area (10) are imaged in the imaging area (19), the light and dark area (9, 10) bordering each other linearly such that a shade edge (17) is formed, and that at least parts of the light and dark area (9, 10) and the shade edge (17) are imaged by means of at least one optical detector (8), and color data (12) and surface data (11) are detected from the images, the at least one light source (6), the at least one edge (7) and the at least one optical detector (8) being disposed at known positions on the x-ray device to perform the method.

6. The method according to claim 5, **characterized in that** the color data (12) and surface data (11) are determined from the images which were recorded at several sequential points in time during the at least partial revolution of the system.

7. The method according to claim 5 or 6, **characterized in that** the color data (12) and the surface data (1) are determined from the images that were recorded from at least four different directions.

8. The method according to one of claims 5 to 7, **characterized in that** the color data (12) and surface data (11) are determined from the images that were detected from directions distributed at even angles over the at least partial revolution of the system.

9. The method according to one of claims 5 to 8, **characterized in that** the images for the color data (12) and images for the surface data (11) are recorded sequentially during the at least partial revolution in equivalent increments of time.

10. The method according to one of claims 5 to 9, **characterized in that**, preceding an image or a series of images, a radiograph (13) as well as surface data and color data of a calibration body (14) present in the imaging area (19) of the x-ray device, or introduced for this purpose in the imaging area (19) of the x-ray device, are generated, and the position of the color data (12) and surface data (11) relative to the radiograph (13) is determined.

11. The method according to claim 10, **characterized in that** the color data (12) and surface data (11) are depicted jointly in the radiograph (13).

12. The method according to one of claims 5 to 11, **characterized in that**, preceding an image or a series of images, a radiograph (13) as well as surface data and color data of a calibration body (14) present in the imaging area (19) of the x-ray device, or introduced for this purpose in the imaging area (19) of the x-ray device, are generated, and a color correction is determined.

13. The method according to one of claims 5 to 12, **characterized in that** a geometric model of the surface is generated from at least two images by means of a stereometric method, the surface data being used in the implementation of the stereometric method.

14. The method according to claim 13, **characterized in that** the resolution of the geometric model of the surface generated by means of the stereometric method is greater than the resolution of the surface data.

15. The method according to claim 13 or 14, **characterized in that** only the illuminated area from the images is used for the stereometric method.

## Revendications

1. Dispositif radiologique dentaire pour générer un cliché radiographique (13) d'un patient (16), comprenant une source de rayons X (3) et un détecteur de rayons X (4), qui sont disposés à chaque fois sur un dispositif support (2) et qui peuvent être déplacés au moyen du dispositif support (2) au moins partiellement autour d'un espace de cliché (19) se trouvant entre la source de rayons X (3) et le détecteur de rayons X (4), ainsi qu'au moins une unité d'enregistrement d'images (5) pour enregistrer la surface d'un patient (16) placé dans l'espace de cliché (19), l'unité d'enregistrement d'images présentant au moins une source lumineuse (6), qui génère un faisceau (6') de lumière visible polychromatique dirigé dans l'espace de cliché (19), et au moins un détecteur optique (8), où l'au moins une source lumineuse (6) et l'au moins un détecteur optique (8) sont disposés directement sur le dispositif support (2) et/ou sur la source de rayons X (3) et/ou sur le détecteur de rayons X (4) et peuvent être déplacés ensemble avec la source de rayons X (3) et le détecteur de rayons X (4) autour de l'espace de cliché (19), **caractérisé en ce que** l'unité d'enregistrement d'images (5) présente au moins un bord (7) qui s'avance au moins partiellement entre la source lumineuse (6) et l'espace de cliché (19) dans le faisceau (6') de lumière polychromatique pour générer un bord d'ombre (17) dans l'espace de cliché (19), où l'au moins un bord (7) est disposé directement sur le dispositif support (2) et/ou sur la source de rayons X (3) et/ou sur le détecteur de rayons X (4) et peut être déplacé ensemble avec la source de rayons X (3) et le détecteur de rayons X (4) autour de l'espace de cliché (19) et où les positions relatives de l'au moins une source lumineuse (6), de l'au moins un bord (7) et de l'au moins un détecteur optique (8) sont connues les unes par rapport aux autres.

2. Dispositif radiographique dentaire selon la revendication 1, **caractérisé en ce que** la source lumineuse (6) est une rampe lumineuse linéaire étendue, qui est constituée par une ou plusieurs sources lumineuses individuelles disposées les unes à côté des autres.

3. Dispositif radiologique dentaire selon l'une quelconque des revendications 1 ou 2, **caractérisé en ce que** le détecteur optique enregistre tant des informations de luminosité que des informations de couleur de l'espace de cliché (19) et les fournit en tant que cliché.

4. Dispositif radiologique dentaire selon la revendication 3, **caractérisé en ce que**, dans l'image brute de la caméra, une zone de plus de 50 %, de préférence de plus de 75 % et en particulier de plus de 85 % est exempte de la projection d'ombre provoquée par le bord d'ombre.

5. Procédé pour générer un cliché radiographique (13) d'un patient (16) au moyen d'un dispositif radiologique, dans lequel des rayons X générés au moyen d'un rayonnement X, pendant une rotation au moins partielle d'un système, constitué par une source de rayons X (3) et un détecteur de rayons X (4), autour d'un espace de cliché (19) se trouvant entre la source de rayons X (3) et le détecteur de rayons X (4), par la source de rayons X (3) et traversant l'espace de cliché (19) sont enregistrés par le détecteur de rayons X à partir de différentes directions, **caractérisé en ce que**, simultanément avec la génération du cliché radiographique (13) pendant la rotation au moins partielle du système, on représente au moyen d'une unité d'enregistrement d'images (5) se déplaçant conjointement avec le système, par un faisceau lumineux (6') polychromatique partant d'au moins une source lumineuse (6), au moins un bord (7) dans l'espace de cliché (19) de manière telle qu'il se forme une zone claire (9) et une zone plus foncée (10) dans l'espace de cliché (19), la zone claire et la zone foncée (9, 10) se jouxtant en forme de ligne de manière telle qu'il se forme un bord d'ombre (17), et qu'au moins des parties de la zone claire et de la zone foncée (9, 10) et du bord d'ombre (17) sont enregistrées au moyen d'au moins un détecteur optique (8) et des données de couleur (12) et des données de surface (11) sont déterminées à partir des clichés, où l'au moins une source lumineuse (6), l'au moins un bord (7) et l'au moins un détecteur optique (8) sont disposés en des positions connues sur le dispositif radiologique pour réaliser le procédé.

6. Procédé selon la revendication 5, **caractérisé en ce que** les données de couleur (12) et les données de surface (11) sont déterminées à partir des clichés qui ont été enregistrés à plusieurs moments successifs dans le temps pendant la rotation au moins partielle du système.

7. Procédé selon la revendication 5 ou 6, **caractérisé en ce que** les données de couleur (12) et les données de surface (11) sont déterminées à partir des clichés qui ont été enregistrés à partir d'au moins quatre directions différentes.

8. Procédé selon l'une quelconque des revendications 5 à 7, **caractérisé en ce que** les données de couleur (12) et les données de surface (11) sont déterminées à partir de clichés qui ont été enregistrés à partir de directions réparties régulièrement d'un point de vue de l'angle sur la rotation au moins partielle du système.

9. Procédé selon l'une quelconque des revendications 5 à 8, **caractérisé en ce que** les clichés pour les données de couleur (12) et les clichés pour les données de surface (11) sont enregistrés consécutivement à des intervalles identiques dans le temps pendant la rotation au moins partielle.

10. Procédé selon l'une quelconque des revendications 5 à 9, **caractérisé en ce qu'**on génère, avant un cliché ou une série de clichés, un cliché radiographique (13) ainsi que des données de surface (11) et des données de couleur (12) d'un corps d'étalonnage (14) présent dans l'espace de cliché (19) du dispositif radiographique ou introduit à cette fin dans l'espace de cliché (19) du dispositif radiographique et on détermine la position relative des données de couleur (12) et des données de surface (11) par rapport au cliché radiographique (13).

11. Procédé selon la revendication 10, **caractérisé en ce que** les données de couleur (12) et les données de surface (11) sont représentées ensemble avec le cliché radiographique (13)

12. Procédé selon l'une quelconque des revendications 5 à 11, **caractérisé en ce qu'**on génère, avant un cliché ou une série de clichés, un cliché radiographique (13) ainsi que des données de surface (11) et des données de couleur (12) d'un corps d'étalonnage (14) présent dans l'espace de cliché (19) du dispositif radiographique ou introduit à cette fin dans l'espace de cliché (19) du dispositif radiographique et on détermine une correction de couleur.

13. Procédé selon l'une quelconque des revendications 5 à 12, **caractérisé en ce qu'**on génère un modèle géométrique de la surface à partir d'au moins deux clichés au moyen d'un procédé stéréométrique, les données de surface étant utilisées lors de la réalisation du procédé stéréométrique.

14. Procédé selon la revendication 13, **caractérisé en ce que** la résolution du modèle géométrique de la surface généré au moyen du procédé stéréométrique est supérieure à la résolution des données de surface.

15. Procédé selon la revendication 13 ou 14, **caractérisé en ce qu'**on utilise, à partir des clichés, uniquement la zone éclairée pour le procédé stéréométrique.
